# EUROPEAN PATENT APPLICATION

(11) **EP 1 944 056 A1**
(43) Date of publication of application: **16.07.2008**
(21) Application number: 06810514.7
(22) Date of filing: 25.09.2006
(51) Int. Cl.: A61N 1/05, A61N 1/39

(54) **MYOCARDIAL ELECTRODE**

(30) Priority: 26.09.2005 JP 2005277635
(71) Applicant: JMS Co., Ltd., Hiroshima-shi, Hiroshima 730-8652 (JP)
(72) Inventor: TANABE, Hiroaki, Tokyo 1060032 (JP); HAYASHI, Shuro, Hiroshima-shi, Hiroshima 7308652 (JP)
(74) Representative: Zinnecker, Armin
(86) International application number: PCT/JP2006/318946
(87) International publication number: WO 2007/034938

(57) **Abstract**

Two through holes are formed in a first electrode part **10** to penetrate the first electrode part **10** in the extending direction of a substrate **30.** The same two through holes are also formed in a second electrode part **20.** A first lead **40** is inserted in and connected to one of the through holes of the first electrode part **10.** A second lead **50** is inserted in the other through hole of the first electrode part **10** while it is insulated from the first electrode part **10** and engaged with an opening edge of the other through hole. An end of the second lead **50** is inserted in and connected to one of the through holes of the second electrode part **20.** An interval between the first electrode part **10** and the second electrode part **20** is determined by the second lead **50.**

## Description

### TECHNICAL FIELD

In a cardiac surgery involving thoracotomy, such as a coronary artery bypass surgery, arrhythmia caused by ventricular and atrial fibrillation is likely to occur. Therefore, electrical stimulation is given to cardiac muscle for defibrillation. In such a case, for example, a myocardial electrode disclosed by Patent Literature 1 may be used. The myocardial electrode of Patent Literature 1 includes two leads connected to positive and negative electrodes of an external cardiac pacemaker, two electrode parts connected to the distal ends of the leads and a substrate supporting the electrode parts at an interval from each other. The substrate is made of insulating material such as silicone rubber and embedded portions of the two electrode parts are embedded in the substrate.

When the cardiac pacemaker is actuated with the conductive surfaces of the electrode parts exposed on the substrate in contact with the surface of the heart, current from the cardiac pacemaker flows into the cardiac muscle through the leads and the electrode parts. As a result, electrical stimulation is given to the cardiac muscle and the defibrillation is carried out.

The myocardial electrode is also used to give the electrical stimulation to the cardiac muscle when cardiac motion deteriorates during a beating heart surgery without using a pump-oxygenator such that the rhythm of the cardiac motion returns to and remains normal. Further, the myocardial electrode is also used in an arrested heart surgery using the pump oxygenerator to restart the heartbeat by giving the electrical stimulation to the cardiac muscle.

For the manufacture of the myocardial electrode, the substrate in which the embedded portions the two electrode parts are embedded is formed by so-called insert molding. Specifically, the two electrode parts are placed in a cavity of a substrate mold and then a substrate material such as a liquid resin is poured in the cavity and cured.
[Patent Literature 1] Japanese Unexamined Patent Publication No. 2004-89384

### DISCLOSURE OF THE INVENTION

### THE PROBLEM THAT THE INEVNTION IS TO SOLVE

In the manufacture of the substrate in which the embedded portions of the two electrode parts are embedded in the manner disclosed by Patent Literature 1, the two electrode parts placed in the cavity of the mold may be misaligned from the predetermined positions by the flow of the resin poured into the cavity. If the substrate material is cured with the electrode parts misaligned from the desired positions, the interval between the two electrode parts of the myocardial electrode may become too small or too large as compared with the predetermined interval. In such a case, desired electrical stimulation cannot be given to the heart. Thus, effective defibrillation cannot be performed and the return to the normal rhythm and the restart of the heartbeat of the arrested heart become difficult.

As a solution to this, the two electrode parts are kept still in the substrate mold to prevent the misalignment from the predetermined positions. In this case, an additional structure for keeping the electrode parts still has to be provided in the substrate mold. Therefore, the configuration of the substrate mold is complicated and the cost of the mold increases. This leads to an increase of the cost of the myocardial electrode.

Under these circumstances, the present invention has been achieved. In the manufacture of the substrate in which the embedded portions of the electrode parts are embedded, an object of the invention is to make it possible to determine the interval between the electrode parts using the existing leads connecting the cardiac pacemaker and the electrode parts such that the predetermined interval is kept between the electrode parts without complicating the configuration of the substrate mold and the cost of the myocardial electrode is reduced.

### MEANS OF SOLVING THE PROBLEM

According to the present invention, the object is achieved by fixing a lead connected to a second electrode part while it is insulated from a first electrode part and determining the interval between the first and second electrode parts by the lead.

Specifically, according to a first aspect of the invention, a myocardial electrode includes: a first electrode part and a second electrode part; a first lead and a second lead for connecting the first electrode part and the second electrode part to a cardiac pacemaker, respectively; and an insulating substrate in which a first embedded portion of the first electrode part and a second embedded portion of the second electrode part are embedded, wherein the first electrode part and the second electrode part are arranged at an interval from each other while conductive surfaces thereof are exposed on the substrate and part of the second lead close to the electrode part is connected to the second embedded portion and fixed to and insulated from the first embedded portion and the interval between the first electrode part and second electrode part is determined by the part of the second lead close to the electrode part.

With this configuration, the second lead is connected to the second embedded portion and integrated with the second electrode part, while the second lead is fixed to and insulated from the first embedded portion and integrated with the first electrode part. The interval between the first and second electrode parts is determined by the second lead. Thus, a predetermined interval is kept between the first and second electrode parts by the second lead.

In the manufacture of the substrate, the first and second electrode parts and part of the second lead close to the electrode part are placed in a cavity of a mold for forming the substrate. Then, a substrate material in a liquid state is poured into the cavity. At this time, as the predetermined interval is kept between the first and second electrode parts by the second lead, the interval between the electrode parts does not become too small or too large even if the flow of the material poured into the cavity hits the first and second electrode parts.

According to a second aspect of the invention related to the first aspect, the conductive surfaces of the first electrode part and the second electrode part are exposed only on one surface of the substrate.

With this configuration, the one surface of the substrate is faced toward the heart such that the conductive surfaces of the first and second electrode parts are brought into contact with the heart. The conductive surfaces of the electrode parts do not come into contact with the other body tissue to which the electrical stimulation is unnecessary. Thus, the current of the cardiac pacemaker is allowed to flow into the cardiac muscle only and prevented from flowing into the other body tissue.

According to a third aspect of the invention related to the first aspect, the substrate includes an electrode part support in which the first embedded portion and the second embedded portion are embedded and an extension part extending from the electrode part support to be in contact with body tissue.

With this configuration, when the conductive surfaces of the first and second electrode parts are brought into contact with the heart, the extension part extended from the electrode part support comes into contact with the heart and the body tissue around the heart. Therefore, the electrode part support is stabilized and the conductive surfaces are prevented from coming off the surface of the heart.

According to a fourth aspect of the invention related to the third aspect, a separation guide at which the extension part is separated from the electrode part support is provided between the electrode part support and the extension part of the substrate.

With this configuration, if the extension part of the substrate is unnecessary, it can easily be separated from the electrode part support to downsize the substrate. Therefore, the first and second electrode parts can be placed in a small area in the thoracic cavity.

According to a fifth aspect of the invention related to the fourth aspect, the electrode part support of the substrate is configured to be insertable into a drain for discharging body fluid seeped into a thoracic cavity to the outside.

With this configuration, when the electrode parts and the substrate are left in the thoracic cavity during a follow-up after a cardiac surgery involving thoracotomy and then the myocardial electrode is no longer necessary, the electrode parts and the substrate are taken out of the thoracic cavity through the drain.

According to a sixth aspect of the invention related to the first aspect, the first embedded portion and the second embedded portion are formed to extend in the extending direction of the substrate.

With this configuration, the first and second embedded portions extend in the extending direction of the substrate. Therefore, the first and second electrode parts are supported on the substrate with stability.

According to a seventh aspect of the invention related to the first aspect, a through hole is formed in the first embedded portion to penetrate the first embedded portion in the extending direction of the substrate and the part of the second lead close to the electrode part is inserted in the through hole and engaged with an opening edge of the through hole to be fixed to the first embedded portion.

With this configuration, the second lead is fixed to the first embedded portion by passing the second lead through the first embedded portion.

### EFFECT OF THE INVENTION

According to the first aspect of the invention, the second lead is connected to the second embedded portion of the second electrode part and fixed to and insulated from the first embedded portion such that the interval between the electrode parts is determined by the second lead. Therefore, the predetermined interval is kept between the first and second electrode parts without complicating the configuration of the mold for forming the substrate in which the first and second embedded portions are embedded. Thus, the mold of the substrate becomes less expensive and the cost is reduced.

According to the second aspect of the invention, the conductive surfaces of the first and second electrode parts are exposed only on one surface of the substrate. Therefore, only the cardiac muscle is electrically stimulated, while the other body tissue to which the electrical stimulation is unnecessary is not electrically stimulated. Thus, the therapy is carried out less invasively.

According to the third aspect of the invention, the extension part extending from the electrode part support is brought into contact with the body tissue to stabilize the electrode part support. Therefore, the conductive surfaces are prevented from coming off the surface of the heart and the electrical stimulation is surely given to the cardiac muscle.

According to the fourth aspect of the invention, the separation guide is provided between the electrode part support and the extension part. This makes it possible to easily separate the extension part from the electrode part support to downsize the substrate. Therefore, the electrode parts are easily and quickly placed in a small area in the thoracic cavity.

According to the fifth aspect of the invention, the electrode parts and the substrate left in the thoracic cavity are taken out of the thoracic cavity through the drain for discharging body fluid from the thoracic cavity without making a thoracoabdominal incision. Therefore, the therapy is carried out less invasively.

According to the sixth aspect of the invention, the first and second embedded portions extend in the extending direction of the substrate. Therefore, the first and second electrode parts are supported on the substrate with stability and the electrical stimulation is surely given to the cardiac muscle.

According to the seventh aspect of the invention, the second lead is inserted in the through hole of the first embedded portion and engaged with the opening edge of the through hole to be fixed to the first embedded portion. Thus, the second lead is arranged within the first embedded portion and the first electrode part and the second lead are made compact.

### BRIEF DESCRIPTION OF DRAWINGS

[FIG. **1**]

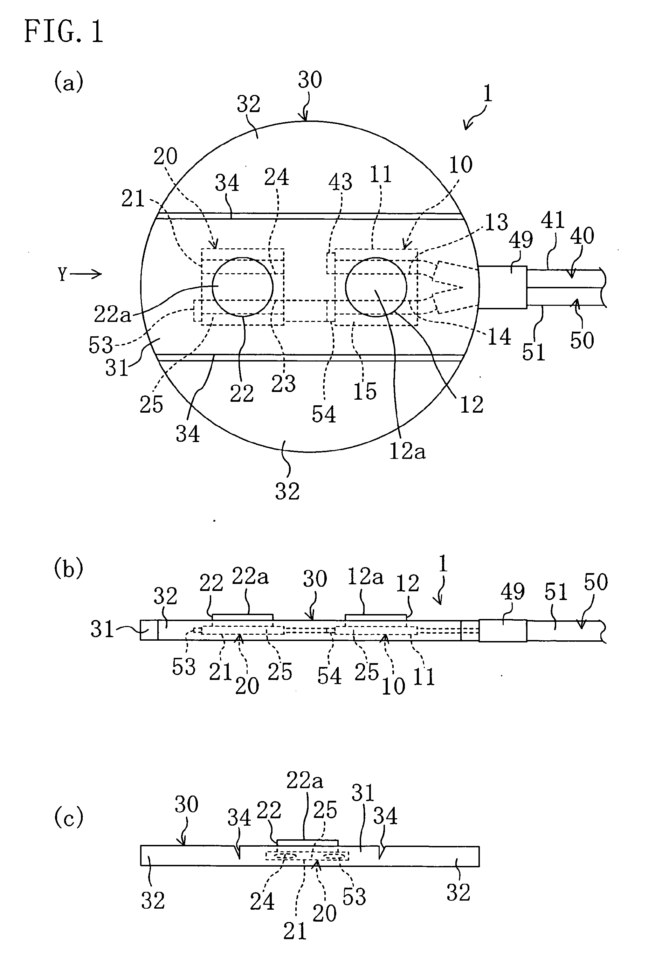

   FIGS. **1A** to **1C** illustrate a myocardial electrode of the present invention. FIG. **1A** is a plan view, FIG. **1B** is a side view and FIG. **1C** is a view observed in the direction of an arrow Y.
[FIG. **2**]

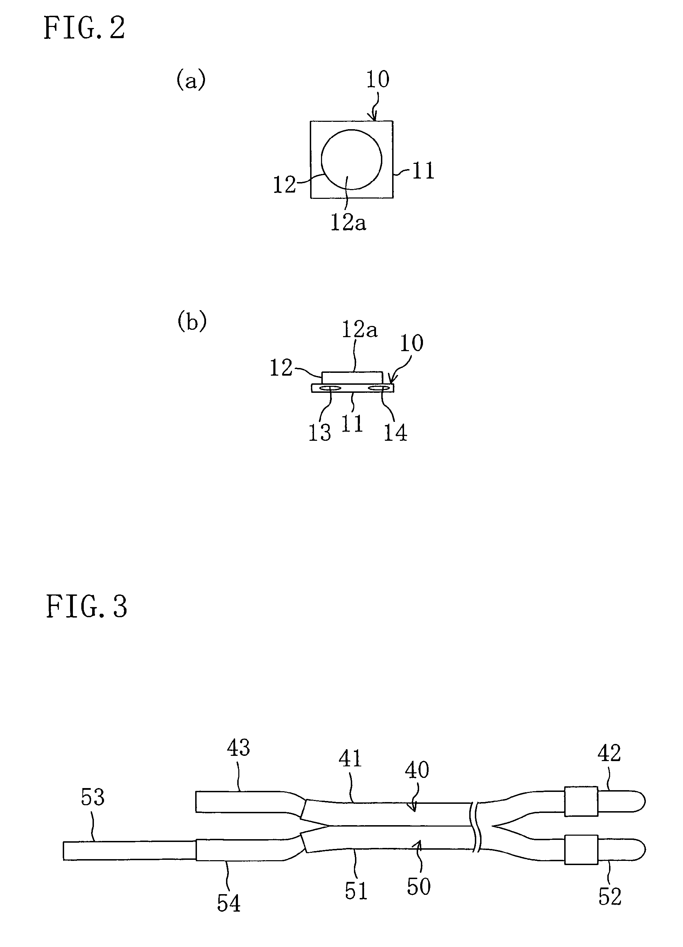

   FIGS. **2A** and **2B** illustrate a first electrode part. FIG. **2A** is a plan view and FIG. **2B** is a side view observed from the side on which through holes are opened.
[FIG. **3**]
   FIG. **3** is a plan view illustrating a first lead and a second lead.
[FIG. **4**]

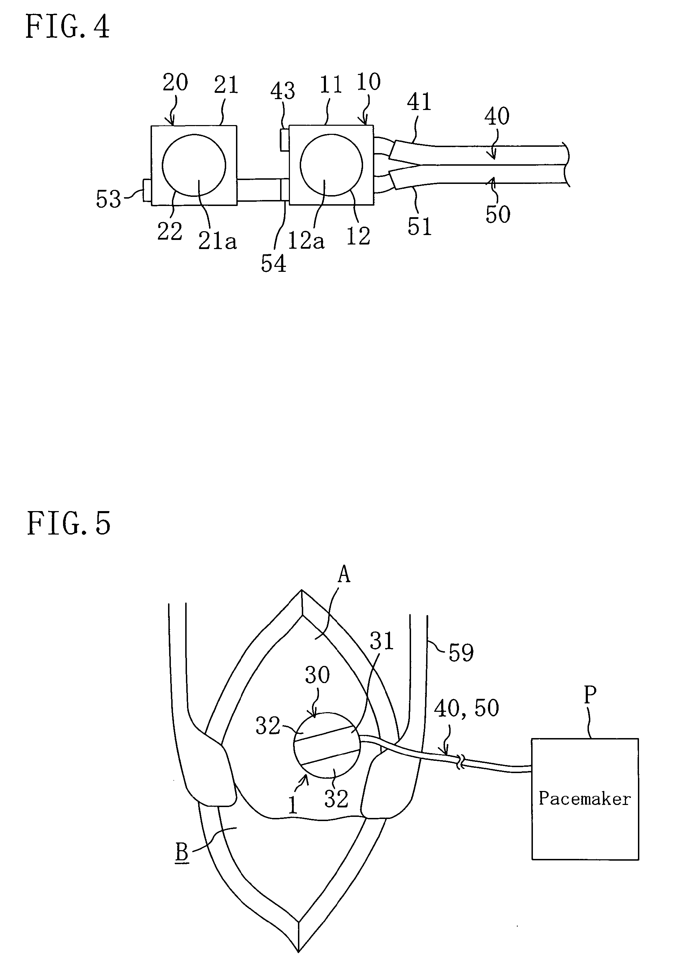

   FIG. **4** is a plan view illustrating the first lead and the second lead connected to a first electrode part and a second electrode part, respectively.
[FIG. **5**]
   FIG. **5** is a view illustrating how the myocardial electrode is used in a coronary artery bypass surgery on a beating heart.
[FIG. **6**]

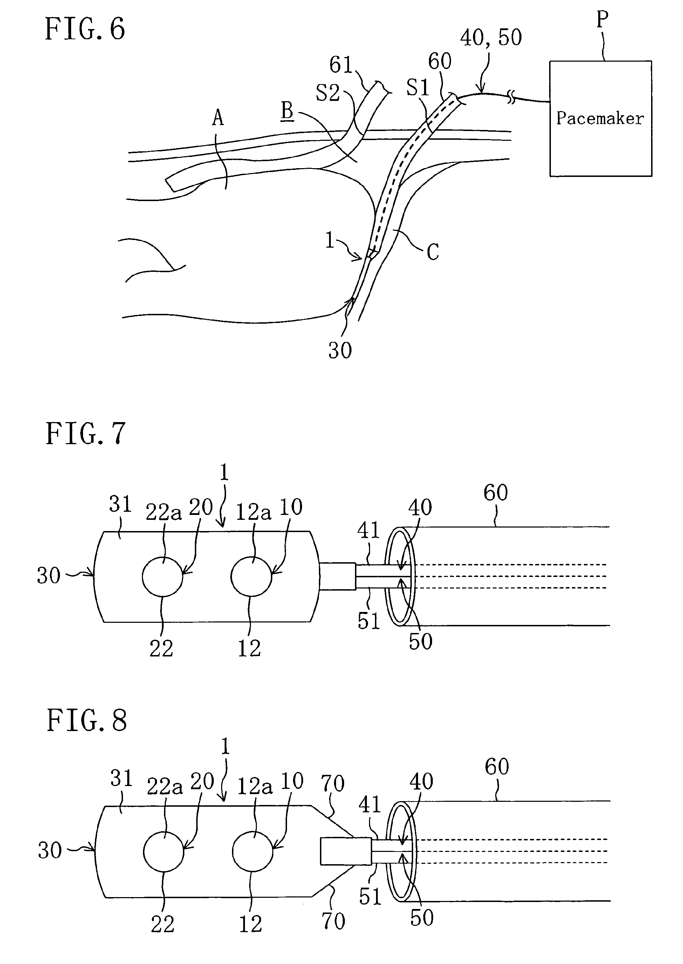

   FIG. **6** is a view illustrating how the myocardial electrode is used after the coronary artery bypass surgery.
[FIG. **7**]
   FIG. **7** is a plan view illustrating the substrate protruding from an end of a first drain.
[FIG. **8**]
   FIG. **8** is a view corresponding to FIG. **7** illustrating a first modification of the embodiment.
[FIG. **9**]

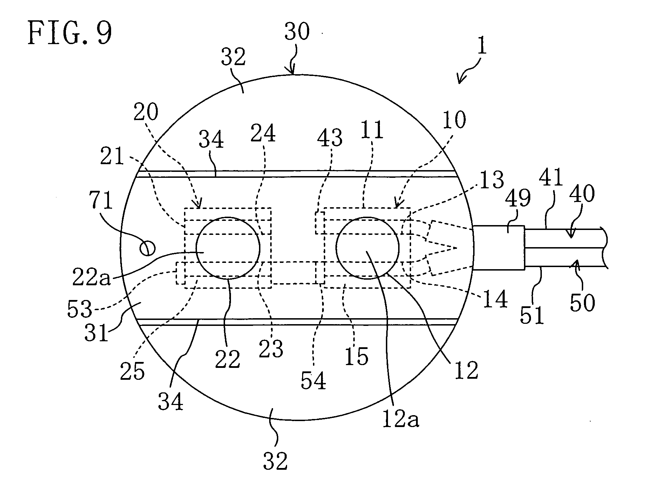

   FIG. **9** is a view corresponding to FIG. **1A** illustrating a second modification of the embodiment.
[FIG. **10**]

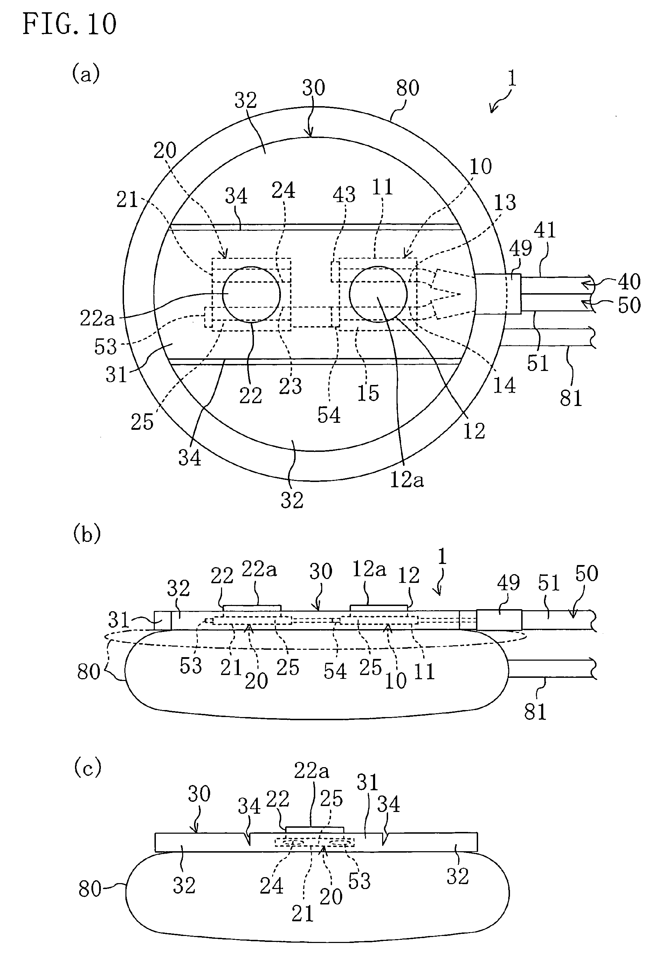

   FIGS. **10A** to **10C** are views corresponding to FIGS. **1A** to **1C** illustrating a third modification of the embodiment.
[FIG. **11**]

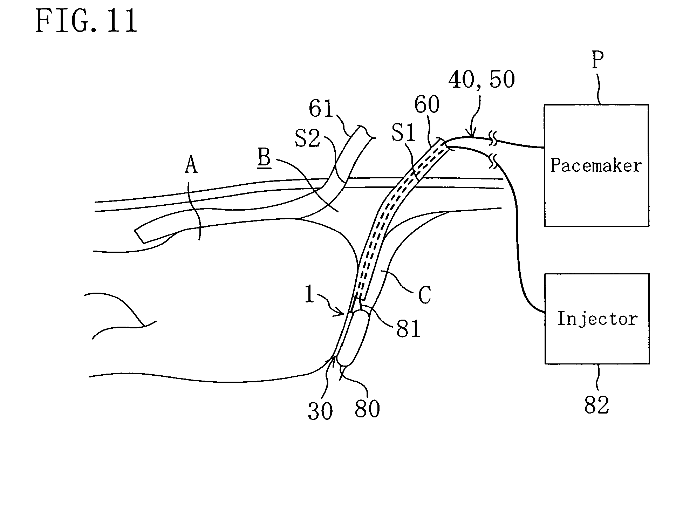

   FIG. **11** is a view corresponding to FIG. **6** illustrating a third modification of the embodiment.
[FIG. **12**]

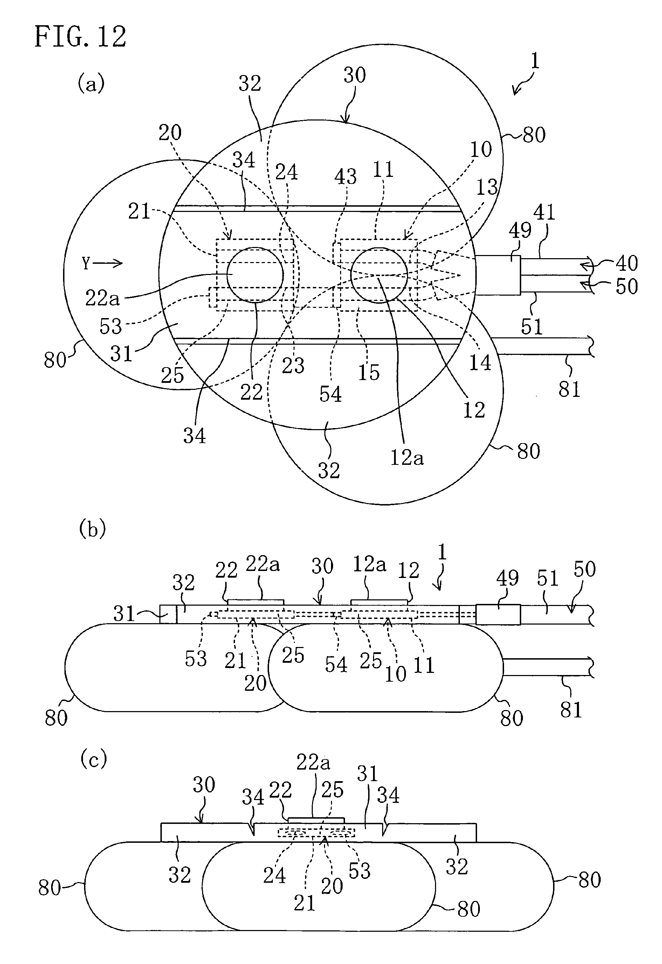

   FIGS. **12A** to **12C** are views corresponding to FIGS. **1A** to **1C** illustrating a fourth modification of the embodiment.
[FIG. **13**]

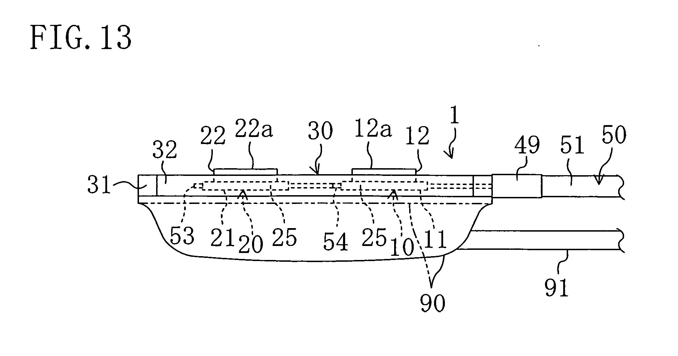

   FIG. **13** is a view corresponding to FIG. **1B** illustrating a fifth modification of the embodiment.

### EXPLANATION OF REFERENCE NUMERALS

- 1: Myocardial electrode
- 10: First electrode part
- 14: Through hole
- 15: First embedded portion
- 20: Second electrode part
- 25: Second embedded portion
- 30: Substrate
- 31: Electrode part support
- 32: Extension part
- 34: Groove (separation guide)
- 40: First lead
- 50: Second lead
- 60: First drain
- P: Cardiac pacemaker

### BEST MODE FOR CARRYING OUT THE INVENTION

Hereinafter, embodiments of the present invention will be described with reference to the drawings. The following embodiments are given for illustrative purpose only and do not intend to limit the present invention, application range and use thereof.

FIGS. **1A** to **1C** show a myocardial electrode **1** of an embodiment of the present invention. The myocardial electrode **1** is used, for example, to give electrical stimulation to cardiac muscle to stop ventricular and atrial fibrillation which occurs during or after a coronary artery bypass surgery. The myocardial electrode **1** is also used to give electrical stimulation to the cardiac muscle when cardiac motion deteriorates during a beating heart surgery without using a pump-oxygenator (not shown) such that the rhythm of the cardiac motion returns to and remains normal. Further, the myocardial electrode **1** is also used in an arrested heart surgery using the pump oxygenerator to restart the heartbeat by giving the electrical stimulation to the cardiac muscle.

The myocardial electrode **1** includes a first electrode part **10,** a second electrode part **20,** a substrate **30** for supporting the first and second electrode parts **10** and **20** at an interval from each other and first and second leads **40** and **50** for connecting the first and second electrode parts **10** and **20** to a cardiac pacemaker **P** (shown in FIGS. **5** and **6**), respectively. The cardiac pacemaker **P** to which the myocardial electrode **1** is connected is an external pacemaker which has widely been used in the medical field and configured to apply a desired pulse current.

As shown in FIGS. **2A** and **2B****,** the first electrode part **10** includes a plate-like rectangular part **11** and a plate-like round part **12** protruding from one surface of the rectangular part **11.** The rectangular part **11** and the round part **12** are integrated into one piece. The thickness of the rectangular part **11** is smaller than that of the substrate **30** as shown in FIGS. **1B** and **1C****.** The rectangular part **11** and a portion of the round part **12** close to the rectangular part **11** are embedded in the substrate **30** as a first embedded portion **15.** The rectangular part **11** embedded in the substrate **30** is arranged to extend in the extending direction of the substrate 30.

In the rectangular part **11,** two through holes **13** and **14** extending in the extending direction of the rectangular part **11** are formed at an interval from each other to have openings on the vertical sides of the rectangular part **11.** The through holes **13** and **14** have the same shape and their inner diameter is determined such that parts of the first and second leads **40** and **50** close to the electrode parts can be inserted therein.

The external size of the round part **12** is set smaller than that of the rectangular part **11.** The round part **12** is located substantially on the center of the one surface of the rectangular part **11.** Therefore, when the first electrode part **10** is viewed in plan, the rectangular part **11** protrudes from the circumference of the round part **12.** As the rectangular part **11** is formed larger than the round part **12** and configured to extend in the extending direction of the substrate **30,** the first electrode part **10** is supported on the substrate **30** with stability.

With the first embedded portion **15** embedded in the substrate **30,** a portion of the round part **12** opposite the rectangular part **11** is exposed and protrudes from only the one surface of the substrate **30.** The portion of the round part **12** protruding from the substrate **30** serves as a conductive surface **12a.** The second electrode part **20** is configured in the same manner as the first electrode part **10.** That is, it includes a rectangular part **21** having two through holes **23** and **24** and a round part **22** including a conductive surface **22a.** The rectangular part **21** and a portion of the round part **22** close to the rectangular part **21** are embedded in the substrate **30** as a second embedded portion **25.**

As shown in FIG. **3****,** the first lead **40** is prepared by covering a conductor formed of a strand of thin steel wires with a coating **41** such as a resin. A connection part **42** (shown in FIG. **3** only) for connection to a positive terminal of the cardiac pacemaker **P** is provided at an end of the first lead **40.** The other end of the first lead **40** close to the electrode part is embedded in the substrate **30** together with the rectangular part **11** of the first electrode part **10** as shown in FIG. **1****.** A cylindrical metallic cover **43** for covering the conductor is fixed onto the end of the first lead **40** close to the electrode part. The dimension of the cover **43** in the direction of the center line is set longer than that of the through holes **13** and **14** of the first electrode part **10.** The end of the first lead **40** close to the electrode part is inserted in the through hole **13** of the first electrode part **10** with the cover **43** fixed thereon. The first electrode part **10** is electrically connected to the conductor via the cover **43.**

With the end of the first lead **40** inserted in the through hole **13,** the ends of the cover **43** in the direction of the center line protrude from the openings of the through hole **13.** Part of the cover **43** protruding from the through hole **13** is crushed such that its width becomes larger than that of the opening of the through hole **13.** Therefore, the cover **43** is engaged with an opening edge of the through hole **13.** As a result, the end of the first lead **40** close to the electrode part is prevented from coming off the through hole **13.**

Just like the first lead **40,** the second lead **50** is prepared by covering a conductor with a coating **51** and a connection part **52** for connection to a negative terminal of the cardiac pacemaker **P** is provided at an end thereof as shown in FIG. **3****.** The other end of the second lead **50** close to the electrode part extends longer than the end of the first lead **40** close to the electrode part and embedded in the substrate **30** together with the rectangular part **21** of the second electrode part **20.** A cover **53** similar to the cover **43** of the first lead **40** is provided at the end of the second lead **50** close to the electrode part. With the end of the second lead **50** close to the electrode part inserted in the through hole **23** of the second electrode part **20,** the cover **53** is engaged with an opening edge of the through hole **23.** Reference numeral **49** in FIG. **1** indicates a lead cover made of a resin.

Part of the second lead **50** closer to the connection part **52** than to the cover **53** is covered with an insulating element **54** different from the coating **51.** The outer diameter of the insulating element **54** is larger than the inner diameter of the through hole **14** of the first electrode part **10.** When the part of the second lead **50** covered with the insulating element **54** is inserted in the through hole **14,** the insulating element **54** is engaged with the opening edge of the through hole **14** such that the second lead **50** is fixed to the first electrode part **10.** Specifically, the first and second electrode parts **10** and **20** are integrated into one piece by the second lead **50** and the interval between the first and second electrode parts **10** and **20** is determined by optionally adjusting the positions of the electrode parts **10** and **20** with respect to the second lead **50.** The through hole **24** of the second electrode part **20** is opened at both ends.

The substrate **30** is made of an insulating material such as silicone rubber and disc-shaped as shown in FIGS. **1A** to **1C**. The substrate **30** includes a narrow electrode part support **31** extending along the diameter of the substrate **30** to pass the center of the substrate **30** and two extension parts **32** extending outward from the lengthwise sides of the electrode part support **31,** respectively. The first embedded portion **15** and the second embedded portion **25** are embedded in the electrode part support **31** at an interval from each other in the lengthwise direction of the electrode part support **31.** A groove **34** is provided between the electrode part support **31** and each of the extension parts **32.** The groove **34** has a substantially V-shaped cross section which is opened on one side of the substrate **30** as shown in FIG. **1C** and functions as a separation guide at which the extension parts **32** are separated from the electrode part support **31.**

Now, the manufacture of the thus-configured myocardial electrode **1** will be described. First, part of the first lead **40** close to the electrode part is inserted in the through hole **13** of the first electrode part **10** and fixed to the first electrode part **10.** Part of the second lead **50** close to the electrode part is inserted in the through hole **14** of the first electrode part **10** and part of the second lead **50** covered with the insulating element **54** is placed in the through hole **14** and engaged with the opening edge of the through hole **14.** As a result, the second lead **50** is fixed to and insulated from the first embedded portion **15.** Then, part of the second lead **40** close to the electrode part is inserted in the through hole **23** of the second electrode part **20** to be fixed to the second electrode part **20.** The interval between the first and second electrode parts **10** and **20** is determined such that desired electrical stimulation is given to the cardiac muscle.

Then, the first and second electrode parts **10** and **20** are placed in a cavity of a mold (not shown) for forming the substrate **30** together with the parts of the first and second leads **40** and **50** close to the electrode part. After the mold is closed, a material of the substrate **30** in a liquid state is poured into the cavity. Then, the flow of the material hits the first and second electrode parts **10** and **20.** As the predetermined interval is kept between the first and second electrode parts **10** and **20** by the second lead **50,** the interval between the first and second electrode parts **10** and **20** is prevented from becoming too small or too large. After the material in the cavity is cured, the mold is opened and the substrate **30** is released from the mold. Thus, the myocardial electrode **1** including the substrate **30** in which the embedded portions **15** and **25** of the first and second electrode parts **10** and **20** are embedded is provided by insert molding.

Use of the myocardial electrode **1** in a coronary artery bypass surgery on a beating heart will be described with reference to FIG. **5****.** Reference numeral **59** in FIG. **5** indicates a rib spreader for keeping an incision in the thorax open. First, as the substrate **30** is placed on a heart **A** with one surface facing the heart **A,** the conductive surfaces **12a** and **22a** come into contact with the heart **A.** At the same time, the extension parts **32** extending from the electrode part support **31** of the substrate **30** are also in contact with the heart **A.** Therefore, the electrode part support **31** remains stable while the heart is beating and the conductive surfaces **12a** and **22a** are prevented from coming off the heart **A.**

When the cardiac pacemaker **P** is actuated, current from the cardiac pacemaker **P** flows into the first and second electrode parts **10** and **20** through the first and second leads **40** and **50,** respectively, and electrical stimulation is given to the cardiac muscle by the first and second electrode parts **10** and **20.** Since the predetermined interval is kept between the first and second electrode parts **10** and **20** as described above, desired electrical stimulation is given to the cardiac muscle and defibrillation is effectively performed.

Further, as the conductive surfaces **12a** and **22a** of the first and second electrode parts **10** and **20** are exposed only on the one surface of the substrate **30,** the conductive surfaces **12a** and **22a** are brought into contact with only a required part of the heart A and do not come in contact with other body tissue to which the electrical stimulation is unnecessary. Thus, the current of the cardiac pacemaker **P** is allowed to flow into the cardiac muscle only and prevented from flowing into the other body tissue.

The first and second electrode parts **10** and **20** are easily placed in a small area in a thoracic cavity **B** by downsizing the substrate **30** by separating the extension parts **32** from the electrode part support **31.** In this case, both or one of the two extension parts **32** may be separated from the electrode part support **31.**

Next, use of the myocardial electrode **1** after the coronary artery bypass surgery will be described with reference to FIGS. **6** and **7****.** After the coronary artery bypass surgery, body fluid seeped into the thoracic cavity **B** is discharged out of the thoracic cavity **B** using a first drain **60** and a second drain **61.** The drains **60** and **61** are circular tubes made of a resin. The width of the electrode part support **31** of the substrate **30** is set smaller than the inner diameter of the drains **60** and **61** such that the electrode part support **31** can be inserted in the drains **60** and **61.**

The first drain **60** is arranged such that an end thereof is inserted between the heart **A** and a diaphragm **C** and the other end protrudes out of the thoracic cavity **B.** The second drain **61** is arranged such that an end thereof is inserted in part of the thoracic cavity **B** above the heart **A** and the other end protrudes out of the thoracic cavity **B.**

In this case, as shown in FIG. **7****,** both of the extension parts **32** of the substrate **30** are separated from the electrode part support **31** and the remaining substrate **30** is inserted in the first drain **60** such that it protrudes from an end of the first drain **60.** After the surgery, the first drain **60** and the substrate **30** is inserted between the heart **A** and the diaphragm **C** while the other end of the first drain **60** protrudes out of the thoracic cavity **B** through an incision **S1.** At the same time, the substrate **30** is sandwiched and fixed between the heart **A** and the diaphragm **C** with the conductive surfaces **12a** and **22a** facing the heart **A.** Thus, the conductive surfaces **12a** and **22a** are brought into contact with the heart **A.** The second drain **61** is also inserted in the thoracic cavity **B** at one end while it protrudes out of the thoracic cavity **B** through an incision **S2** at the other end. Reservoir bags (not shown) for keeping the body fluid discharged from the thoracic cavity **B** are attached to the other ends of the first and second drains **60** and **61,** respectively.

When the cardiac pacemaker **P** is actuated, current from the cardiac pacemaker **P** flows into the cardiac muscle. As the predetermined interval is kept between the first and second electrode parts **10** and **20,** the defibrillation is effectively performed. Further, since the conductive surfaces **12a** and **22a** are exposed only on the one surface of the substrate **30,** the electrical stimulation is not given to the diaphragm **C.**

When the condition of the patient becomes stable after the surgery, the first and second electrode parts **10** and **20** and the substrate **30** are taken out of the thoracic cavity **B.** For taking them out, the first and second leads **40** and **50** are pulled at the connection parts **42** and **52.** Then, the substrate **30** is drawn into the first drain **60** from the first electrode part **10.** As the first and second leads **40** are **50** pulled more, the first and second electrode parts **10** and **20** and the substrate **30** pass through the first drain **60** and are taken out of the thoracic cavity **B.** Thus, there is no need of making a thoracoabdominal incision to take out the first and second electrode parts **10** and **20** and the substrate **30.**

As described above, the myocardial electrode **1** of the present embodiment is configured such that the second lead **50** connected to the second electrode part **20** is fixed to the first electrode part **10** and the interval between the first and second electrode parts **10** and **20** is determined by the second lead **50.** Therefore, in the manufacture of the substrate **30** in which the first and second embedded portions **15** and **25** are embedded, the predetermined interval is kept between the first and second electrode parts **10** and **20** without complicating the configuration of the mold. Thus, the mold of the substrate **30** becomes less expensive and the cost is reduced.

Since the conductive surfaces **12a** and **22a** of the first and second electrode parts **10** and **20** are exposed only on the one surface of the substrate **30,** only the cardiac muscle is electrically stimulated, while the other body tissue to which the electrical stimulation is unnecessary is not electrically stimulated. Thus, the therapy is carried out less invasively.

The substrate **30** is provided with the electrode part support **31** and the extension parts **32** extending from the electrode part support **31** to be in contact with the body tissue. Therefore, when the conductive surfaces **12a** and **22a** of the first and second electrode parts **10** and **20** are brought into contact with the heart **A,** the extension parts **32** come into contact with the heart **A** and the body tissue around the heart **A** to stabilize the electrode part support **31.** Accordingly, the conductive surfaces **12a** and **22a** are prevented from coming off the surface of the heart **A** and the electrical stimulation is surely given to the cardiac muscle.

With the provision of the grooves **34** between the electrode part support **31** and the extension parts **32** of the substrate **30,** the extension parts **32** are easily separated from the electrode part support **31** to downsize the substrate **30.** Therefore, the first and second electrode parts **10** and **20** can be inserted easily and quickly in a small area in the thoracic cavity **B.**

The electrode part support **31** of the substrate **30** is configured to be insertable in the first drain **60.** Therefore, when the first and second electrode parts **10** and **20** are left in the thoracic cavity **B** during a follow-up after the surgery and then the electrical stimulation is no longer necessary, the first and second electrode parts **10** and **20** and the substrate **30** are taken out without making a thoracoabdominal incision. Thus, the therapy is carried out less-invasively.

The rectangular parts **11** and **21** are formed to extend in the extending direction of the substrate **30.** Therefore, the first and second electrode parts **10** and **20** are supported on the substrate **30** with stability.

The rectangular part **11** is provided with the through hole **14** penetrating the rectangular part **11** in the extending direction of the substrate **30** and part of the second lead **50** close to the electrode part is inserted in the through hole **14** and engaged with the opening edge of the through hole **14** to be fixed the first electrode part **10.** Therefore, the second lead **50** is arranged within the rectangular part **11.** Thus, the first electrode part **10** and the second lead **50** are made compact.

With use of a myocardial electrode of a needle type or an alligator clip type which has conventionally used in a cardiac surgery, the cardiac muscle may be scratched and damaged. In contrast, the myocardial electrode **1** of the present embodiment is used by merely applying the first and second electrode parts **10** and **20** onto the cardiac muscle. Therefore, the electrical stimulation is applied less invasively without causing any damage to the cardiac muscle.

According to a first modification of the embodiment shown in FIG. **8****,** an edge of the electrode part support **31** close to the first electrode part **10** may be provided with a guiding part **70** for guiding the substrate **30** into the first drain **60** when the substrate **30** is drawn into the first drain **60.** The guiding part **70** is configured to protrude from the electrode part support **31** toward the connection parts **42** and **52** of the leads **40** and **50** and tapered toward the connection parts **42** and **52** of the leads **40** and **50** with respect to the center line of the leads **40** and **50.** With this configuration, when the substrate **30** left in the thoracic cavity **B** is taken out through the first drain **60,** the guiding part **70** slides along the opening edge of the first drain **60** such that the substrate **30** is smoothly drawn into the first drain **60.**

According to a second modification of the embodiment shown in FIG. **9****,** a hole **71** may be provided in the vicinity of the edge of the electrode part support **31** to penetrate the electrode part support **31** in the thickness direction. With this configuration, the electrode part support **31** placed between the heart **A** and the diaphragm **C** is fixed to that position by sewing the electrode part support **31** onto the surface tissue of the heart **A** with a thread run through the hole **71.** Thus, the electrode part support **31** is kept at the predetermined position.

According to a third modification of the embodiment shown in FIGS. **10A** to **10C** and **11,** a balloon **80** for pressing the electrode part support **31** against the surface of the heart **A** may be provided on the surface of the electrode part support **31** opposite the conductive surfaces **12a** and **22a.** The balloon **80** is circular and larger than the substrate **30** and adhered to the electrode part support **31** with an adhesive, for example. An end of an air line **81** is connected to the balloon **80** to communicate with the inner space of the balloon **80.** The air line **81** is made of a flexible resin and has a diameter smaller than the inner diameter of the first drain **60.** The other end of the air line **81** is connected to an air injector **82.** The injector **82** may be a syringe, for example.

According to the third modification, the balloon **80** in a deflated state (indicated by a broken line in FIG. **10B**)**.** As shown in FIG. **11****,** the electrode part support **31** are placed between the heart **A** and the diaphragm **C** and then the balloon **80** is inflated by injecting air into the balloon **80** with the injector **82.** As a result, the electrode part support **31** is brought into close contact with the heart **A.** Though not shown, after the balloon **80** is inflated, the air flow may be blocked by pinching the middle part of the air line **81** with a clip or closing a switching valve provided in the middle of the air line **81.** For taking the electrode part support **31** out of the thoracic cavity **B,** the balloon **80** is deflated to shrink the balloon **80.**

According to a fourth modification of the embodiment shown in FIGS. **12A** to **12C**, the electrode part support **31** may be provided with three balloons **80.** In this modification, the inner spaces of the three balloons **80** are communicated with each other and the air line **81** is connected to one of the balloons **80.** With the provision of the three balloons **80,** the electrode part support **31** is brought into close contact with the heart A with stability. Thus, the electrical stimulation is surely given to the cardiac muscle.

According to a fifth modification of the embodiment shown in FIG. **13****,** a thin circular sheet **90** made of silicone rubber may be used in place of the balloon **80.** The periphery of the circular sheet **90** is adhered to the periphery of the substrate **30** with an adhesive and air is introduced between the sheet **90** and the substrate **30** to inflate the sheet **90.** Reference numeral **91** indicates an air line similar to that used in the fourth modification and is connected to an injector.

The electrode part support **31** may be placed not only between the heart **A** and the diaphragm **C** but also between the heart **A** and the body tissue around the heart **A** such as a thoracic wall. Also in this case, the balloon **80** or the sheet **90** may be provided and inflated to keep the electrode part support **31** in close contact with the heart **A.**

The shapes of the first electrode part **10,** the second electrode part **20** and the substrate **30** are not limited to those described above and may optionally be varied. Further, the material of the substrate **30** is not limited to silicone rubber and other kinds of rubbers and resins may be used.

The myocardial electrode **1** can be used also in other surgeries than the cardiac surgery, such as a surgery which requires thoracotomy and may cause fibrillation.

The separation guide provided between the electrode part support **31** and the extension parts **32** of the substrate **30** may be realized by other means than the grooves **34,** such as a plurality of through holes or hollow parts.

### INDUSTRIAL APPLICABILITY

As described above, for example, the myocardial electrode of the present invention is used for stopping ventricular and atrial fibrillation which occurs during a coronary artery bypass surgery.

## Claims

1. A myocardial electrode comprising:
a first electrode part and a second electrode part;
a first lead and a second lead for connecting the first electrode part and the second electrode part to a cardiac pacemaker, respectively; and
an insulating substrate in which a first embedded portion of the first electrode part and a second embedded portion of the second electrode part are embedded, wherein
the first electrode part and the second electrode part are arranged at an interval from each other while conductive surfaces thereof are exposed on the substrate and
part of the second lead close to the electrode part is connected to the second embedded portion and fixed to and insulated from the first embedded portion and the interval between the first electrode part and second electrode part is determined by the part of the second lead close to the electrode part.

2. The myocardial electrode of Claim 1, wherein
the conductive surfaces of the first electrode part and the second electrode part are exposed only on one surface of the substrate.

3. The myocardial electrode of Claim 1, wherein
the substrate includes an electrode part support in which the first embedded portion and the second embedded portion are embedded and an extension part extending from the electrode part support to be in contact with body tissue.

4. The myocardial electrode of Claim 3, wherein
a separation guide at which the extension part is separated from the electrode part support is provided between the electrode part support and the extension part of the substrate.

5. The myocardial electrode of Claim 4, wherein
the electrode part support of the substrate is configured to be insertable into a drain for discharging body fluid seeped into a thoracic cavity to the outside.

6. The myocardial electrode of Claim 1, wherein
the first embedded portion and the second embedded portion are formed to extend in the extending direction of the substrate.

7. The myocardial electrode of Claim 1, wherein
a through hole is formed in the first embedded portion to penetrate the first embedded portion in the extending direction of the substrate and
the part of the second lead close to the electrode part is inserted in the through hole and engaged with an opening edge of the through hole to be fixed to the first embedded portion.
